# EUROPEAN PATENT SPECIFICATION

(11) **EP 1 482 975 B1**
(45) Date of publication and mention of the grant of the patent: **13.12.2006**
(21) Application number: 03702560.8
(22) Date of filing: 29.01.2003
(51) Int. Cl.: A61K 45/00, A61K 41/00, A61K 39/395

(54) **PREVENTION OF TISSUE ADHESION**
VORBEUGUNG VON GEWEBEADHÄSION
PREUENTION D'ADHERENCES DE TISSUS

(30) Priority: 29.01.2002 GB 0201983; 04.02.2002 GB 0202379; 29.10.2002 GB 0225128
(43) Date of publication of application: 08.12.2004
(73) Proprietor: Vlaams Interuniversitair Instituut voor Biotechnologie vzw., 9052 Zwijnaarde (BE); Thromb-X, 3000 Leuven (BE); K.U. Leuven Research and Development, 3000 Leuven (BE)
(72) Inventor: KONINCKX, Philippe, B-3360 Bierbeek (BE); CARMELIET, Peter, B-3052 Blanden (BE); COLLEN, Désiré, London, Greater London SW5 OHN (GB); MOLINAS SANABRIA, Carlos, Roger, B-3000 Leuven (BE)
(74) Representative: Bird, Ariane
(86) International application number: PCT/EP2003/000892
(87) International publication number: WO 2003/063904

(56) References cited:
- WO-A-01/32651
- WO-A-02/36054
- WO-A-98/50064
- WO-A-99/15530
- DATABASE HCA [Online] CHEMICAL ABSTRACTS SERVICE, COLUMBUS, OHIO, US; ZHENG, ZHEN-HUA ET AL: "Prevention of postoperative abdominal adhesions by an antibody to VEGF in mice" retrieved from STN Database accession no. 1999:230979 XP002245693 & SHIJIE HUAREN XIAOHUA ZAZHI (1999), 7(3), 227-229 ,
- SALTZMAN ANDREW K ET AL: "Prevention of postoperative adhesions by an antibody to vascular permeability factor/vascular endothelial growth factor in a murine model." AMERICAN JOURNAL OF OBSTETRICS AND GYNECOLOGY, vol. 174, no. 5, 1996, pages 1502-1506, XP009012766 ISSN: 0002-9378

## Description

### Field of the invention

The present invention is concerned with compositions for use in the medical art. More particularly the invention relates to uses of inhibitors against hypoxia-induced genes for the manufacture of a medicament to prevent and/or to suppress post-operative/post-wounding adhesions formation. Post-operative adhesions are an unwanted result from surgery and are a major source of postoperative morbidity and mortality. The invention applies to human and veterinary applications. To date, no single therapeutic approach has proven universally effective in preventing formation of post-operative adhesions formations.

### Background of the invention

Post-operative adhesion formation (POA) is a frequent surgical complication in gynaecological, pelvic, and cardiological surgeries. Surgical trauma to the tissues often causes permanent scar formation which connects the traumatized tissue to another organ. Thus at the site of such damage, internal tissues that normally remain separate often become joined together. Complications arising form adhesion formation are intestinal obstructions, small bowel obstructions, chronic pelvic pain, and infertility in women. POA occurs both in traditional surgery and in laparoscopic surgery. Exact data on the prevalence and severity of these consequences are not available since adhesions vary with the severity of surgery, and since systematic second look laparoscopies cannot be performed for obvious ethical reasons. Adhesions occur in over 50 % of patients following a laparotomy, whereas the risk of re-intervention because of adhesions following a laparotomy was recently estimated at 35% within 10 years in a large survey in Scotland (Ellis H. et al, Lancet 353:1476-1480, 1999). The morphological events involved in adhesion formation are well known (Holmdahl L et al, Eur J Surg Suppl 56-62, 1997 and DiZerega GS, Eur J Surg Suppl 10-16, 1997). A peritoneal defect will cause exudation, fibrin deposition, followed by an inflammatory reaction, fibrinolysis and complete reepithelialisation within 3 to 8 days. This rapid healing is a consequence of the regeneration of the mesothelial layer from multiple foci in the lesion and not from the borders as is found during repair of another epithelium.

The direct consequence of this is that the duration of reepithelialisation is independent of the denuded area in the peritoneum. If this rapid healing process fails by an overload of fibrin (e.g. through bleeding), by a decreased fibrinolysis (e.g. as a consequence of a more severe tissue trauma), resulting in a persistent fibrin matrix (Bittinger F, J Surg Res 82:28-33, 1999), or by the presence of a prolonged inflammatory reaction (e.g. by an infection or by suture material), this will lead to prolonged fibroblast proliferation, collagen deposition, angiogenesis and ultimately adhesion formation. However, the biochemical and cellular processes involved in the healing process and in adhesion formation are largely unknown (Holmdahl L and Ivarsson ML: Eur J Surg 165:1012-1019, 1999), and this is reflected in the still inadequate clinical prevention of adhesion formation. In animal models postoperative adhesions can be reduced by a variety of agents such as the intraperitoneal application of anti-inflammatory drugs (Rodgers KE Prog Clin Biol Res 358:119-129, 1990), t-PA to increase fibrinolysis, as the introduction of an overload of mesenchymal cells in the peritoneal cavity after surgery (Bertram P et al Eur J Surg 165:705-709, 1999). Adhesion formation can also be modulated by cytokines, as TGF-β and TNF-α (Chegini N et al J Soc Gynecol Investig 6:153-157, 1999), calcium channel blockers, phospholipase a, sodium-carboxymethyl-cellulose, vitamine-E and phosphatidylinositol. Clinically the prevention of adhesion formation is based upon good surgical technique, which consist mainly in avoiding bleeding and tissue trauma either mechanically or by desiccation. Adhesion formation can further be reduced by various approaches such as soluble or mechanical barrier methods (Haney AF and Doty E: Fertil Steril 70:145-151, 1998; Diamond MP Fertil Steril 69:1067-1074, 1998; Sawada T. et al, Hum Reprod 14:1470-1472, 1999; Wiseman DM J Reprod Med 44:325-331, 1999; Thornton MH. Et al Hum Reprod 13:1480-1485, 1998), or by the inhibition of the inflammatory reaction by corticoids and/or non steroidal anti-inflammatory agents (Buckenmaier CC. et al, Am Surg 65:274-282, 1999). In extreme cases, debilitating adhesions can-be treated by adhesiotomy, surgical section or lysis of the adhesion (adhesiolysis). Endoscopic surgery, so-called minimal invasive surgery, has been claimed to cause less postoperative adhesions than a laparotomy but the data to support this are conflicting. The discrepancy in the reported results can be explained to some extend by the duration of surgery which was not taken into account in these studies. Indeed we recently demonstrated that the duration of CO₂ pneumoperitoneum is a major cofactor in adhesion formation in rabbits (Ordonez JL, Dominguez J, Evrard V, Koninckx PR: Hum Reprod 12:2654-2657, 1997; Molinas CR, Koninckx PR: Hum Reprod 15:1758-1763, 2000 and Yesildaglar N, Koninckx PR: Adhesion formation in intubated rabbits increases with high insufflation pressure during endoscopic surgery. Hum Reprod 15:687-691, 2000) and in mice (Molinas CR, Mynbaev O, Pauwels A, Novak P, Koninckx PR: Fertil Steril 76:560-567, 2001 and Yesildaglar N, Ordonez JL, Laermans I, Koninckx PR: Hum Reprod 14:55-59, 1999). Numerous patents are available in the art that claim methods and compositions for the prevention of adhesion formation. In general, the treatments fall into different categories such as (i) (bio)mechanical by avoiding direct contact between tissues, (ii) prevention of fibrin or collagen deposition in the peritoneal exudate or removal of fibrin deposit, (iii) reduction of local tissue inflammation, (iv) inhibition of oxidative damage to tissue, (v) inhibitors of vitronectins or (vi) anoxaemia preventing compounds or combinations thereof. So far none of the existing therapeutic approaches has proven universally effective in preventing postoperative adhesion formation. There is still a need for compositions and methods which can be used safely and effectively to prevent adhesion formation in a variety of different settings. It has been shown in the art that an important factor that causes adhesion formation during surgery is hypoxaemia (Molinas CR and Koninckx PR (2000) Hum. Reprod. 15(8):1758-63). Hypoxia has indeed a dramatic effect on gene expression and micro-array reports described in the art refer to at least 10% of the genes that have a change in expression in the studied systems (Jin K et al (2002) Neurochem. Res. 27(10): 1105-12). In the present invention we have identified several genes which over-expression in hypoxic conditions lead to an enhanced adhesion formation. We have shown that the inhibition of the expression of vascular endothelial growth factor-B (VEGF-B), placental growth factor (PIGF) and Hypoxia inducible factors (HIF) leads to a remarkable suppression of postoperative adhesion formation.

### Figures:

Figure 1: The rabbit and mouse laparoscopic model
Figure 2: Effect of duration of CO₂ pneumoperitoneum upon adhesion formation in non-intubated rabbits (A) and mice (B). Mean ± SEM is indicated together with P value (Wilcoxon).
Figure 3: Effect of insufflation pressure (5 vs. 20 mm of Hg) and flow rate (1 vs. 10 l/min) in intubated rabbits (A) and of insufflation pressure (5 vs. 15 cm of water) and duration of pneumoperitoneum (10 vs. 60 min) in intubated mice (B) upon adhesion formation. Mean ± SEM is indicated together with P value (two-way analysis of variance). (A: Hum Reprod 15, 687-691; B: Fertil Steril, 76, 560-567).
Figure 4: Effect of the addition of different proportions of oxygen to CO₂ pneumoperitoneum upon adhesion formation in rabbits (A) and mice (B). Mean ± SEM is indicated together with P value (Wilcoxon). (B: Fertil Steril, 76, 560-567).
Figure 5: Effect of insufflation gas (CO₂ ■ vs. helium □), duration of pneumoperitoneum (10 vs. 45 min) and addition of oxygen (0 vs. 4%) in rabbits (A) and of insufflation gas (CO2 vs. helium) and addition of oxygen (0 vs. 3%) in mice (B) upon adhesion formation. Mean ± SEM is indicated together with P value (two-way analysis of variance). (A: Hum Reprod 15, 1758-1763; B: Fertil Steril, 76, 560-567)

### Aims and detailed description of the invention

This invention relates to the use of a compound for the manufacture of a medicament for treatment particularly the prevention or suppression) of formation or reformation of adhesions, particularly in the peritoneal or pelvic cavities resulting from wound, surgery, infection, inflammation or trauma. The invention is useful for inhibiting, suppressing or ameliorating adhesion formation in mammals, including humans. The invention applies to human and veterinary applications. The treatments have been shown to be especially effective in preventing adhesion formation in the peritoneum following surgery. In addition, the present invention finds utility in other contexts, e.g., for cardiovascular, orthopedic, thoracic, ophthalmic, CNS and other uses, where prevention of the formation of adhesions is a significant concern.

Some terms and definitions in the present invention will be defined first. The term "adhesion formation" as used herein in its medical sense refers to conglutination, the process of adhering or uniting of two surfaces or parts. For example, the union of the opposing surfaces of a wound, or opposing surfaces of peritoneum. Also, adhesions, in the plural, can refer to inflammatory bands that connect opposing serous surfaces. The term adhesion, as used herein, also includes fibrinous adhesions, which are adhesions that consist of fine threads of fibrin resulting from an exudate of plasma or lymph, or an extravasion of blood. Keloid, a smooth overgrowth of fibroblastic tissue that arises in an area of injury or, occasionally, spontaneously is also a form of adhesion. Basal adhesion formation as used herein in its medical sense is the basal level of adhesion formation that occurs after injury wounding (e.g. surgery) exposed to an atmosphere which contains sufficient oxygen to avoid a condition of hypoxia or of hyperoxia. Under the experimental conditions intubation of small animals with CO2 pneumoperitoneum without oxygen increased levels of postoperative adhesion formation ("hypoxia enhanced adhesion formation"). The term "anoxemia" as used herein means in its medical sense i.e. a decreased availability of oxygen to the cell and the consequences thereof. Anoxemia therefore also comprises hypoxemia, which can be a consequence of decreased oxygen delivery to the cells (e.g. by decreased oxygen concentration in the air or by a failing delivery system such haemoglobin or cardiovascular) or by decreased capacity of the cells to use oxygen.

The term "pharmaceutically acceptable" is used adjectivally herein to mean that the modified noun is appropriate for use in a pharmaceutical product. The term "treatment" refers to any process, action, application, therapy, or the like, wherein a mammal, including a human being, is subject to medical aid with the object of improving the mammal's condition, directly or indirectly. In the current invention "treatment" refers to prevention. When adhesion formation is prevented it means here that the occurrence of adhesion formation is suppressed as compared with the mammal not treated with a PIGF inhibitor of the invention. Suppression means that adhesion formation and more specifically postoperative adhesion formation occurs for at least 20%, 30%, 40%, 50%, 60%, 70%, 80%, 90% or even 100% less than compared with the mammal as compared with the mammal not treated with an inhibitor (e.g. HIF- or PIGF or VEGF-B - inhibitor) of the invention. Adhesion formation can be measured (or scored) as described herein further in the examples.

The invention provides the use of a compound that inhibits the expression and/or activity of a placenta growth factor for the manufacture of a medicament for treatment of adhesion formation. The term 'a compound that inhibits the expression' refers here to gene expression and thus to the inhibition of gene transcription and/or translation of a gene transcript (mRNA) such as for example the PIGF gene or PIGF mRNA. Preferably said inhibition is at least 20%, 30%, 40%, 50%, 60%, 70%, 80%, 90% or even higher. The term ' a compound that inhibits the activity' refers here to the protein that is produced such as the PIGF protein. Said inhibition of activity leads to a diminished interaction (e.g. in the case of PIGF with the VEGFR-1) with its receptor and an inhibition of signal transduction, or a diminished transactivation (as in the case of hypoxia inducible factors which are transcription factors). Preferably said inhibition is at least 20%, 30%, 40%, 50%, 60%, 70%, 80%, 90% or even higher.

The present disclosure shows that adhesion formation is significantly suppressed in adult PIGF^{-/-} mice and that adhesion formation can be suppressed by the usage of inhibitors of PIGF. Thus in one embodiment the present invention relates to the usage of molecules which comprise a region that can specifically bind to placental growth factor or to its receptor (vascular endothelial growth factor receptor-1) and consequently said molecules interfere with the binding of PIGF to its receptor interfering with the signal transduction of PIGF and said molecules can be used for the manufacture of a medicament for treatment of adhesion formation. Thus more specifically the invention relates to molecules that neutralize the activity of PIGF by interfering with its synthesis, translation, dimerisation, receptor-binding and/or receptor-binding-mediated signal transduction. By molecules it is meant peptides, tetrameric peptides, proteins, organic molecules, mutants of the VEGFR-1, soluble receptors of VEGFR-1 and any fragment or homologue thereof having the same neutralizing effect as stated above. Also, the invention the molecules comprise antagonists of PIGF such as anti-PIGF antibodies and functional fragments derived thereof, anti-sense RNA and DNA molecules and ribozymes that function to inhibit the translation of PIGF, all capable of interfering/or inhibiting the VEGFR-1 signal transduction. By synthesis it is meant trancription of PIGF. Small molecules can bind on the promoter region of PIGF and inhibit binding of a transcription factor or said molecules can bind said transcription factor and inhibit binding to the PIGF-promoter. By PIGF it is meant also its isoforms, which occur as a result of alternative splicing, and allelic variants thereof. As a result of alternative splicing, three PIGF RNAs encoding monomeric human PIGF-1, PIGF-2 and PIGF-3 isoform precursors containing 149, 179 and 219 amino acid residues, respectively, have been described. In normal mouse tissues, only one mouse PIGF mRNA encoding the equivalent of human PIGF-2 has been identified. Suitable inhibitors of PIGF described in WO0185796 can also be used for the manufacture of a medicament for treatment of (post-operative) adhesion formation.

The term 'antibody' or 'antibodies' relates to an antibody characterized as being specifically directed against PIGF, VEGFR-1, or any functional derivative thereof, with said antibodies being preferably monoclonal antibodies; or an antigen-binding fragment thereof, of the F(ab')₂, F(ab) or single chain Fv type, or any type of recombinant antibody derived thereof. These antibodies of the invention, including specific polyclonal antisera prepared against PIGF, VEGFR-1, or any functional derivative thereof, have no cross-reactivity to others proteins. The monoclonal antibodies of the invention can for instance be produced by any hybridoma liable to be formed according to classical methods from splenic cells of an animal, particularly of a mouse or rat immunized against PIGF, VEGFR-1, or any functional derivative thereof, and of cells of a myeloma cell line, and to be selected by the ability of the hybridoma to produce the monoclonal antibodies recognizing PIGF, VEGFR-1, or any functional derivative thereof which have been initially used for the immunization of the animals. The monoclonal antibodies according to this embodiment of the invention may be humanized versions of the mouse monoclonal antibodies made by means of recombinant DNA technology, departing from the mouse and/or human genomic DNA sequences coding for H and L chains or from cDNA clones coding for H and L chains. Alternatively the monoclonal antibodies according to this embodiment of the invention may be human monoclonal antibodies. Such human monoclonal antibodies are prepared, for instance, by means of human peripheral blood lymphocytes (PBL) repopulation of severe combined immune deficiency (SCID) mice as described in PCT/EP 99/03605 or by using transgenic non-human animals capable of producing human antibodies as described in US patent 5,545,806. Also fragments derived from these monoclonal antibodies such as Fab, F(ab)'₂ and ssFv ("single chain variable fragment"), providing they have retained the original binding properties, form part of the present invention. Such fragments are commonly generated by, for instance, enzymatic digestion of the antibodies with papain, pepsin, or other proteases. It is well known to the person skilled in the art that monoclonal antibodies, or fragments thereof, can be modified for various uses. The antibodies involved in the invention can be labeled by an appropriate label of the enzymatic, fluorescent, or radioactive type.

Small molecules, e.g. small organic molecules, and other drug candidates can be obtained, for example, from combinatorial and natural product libraries.

Random peptide libraries, such as the use of tetrameric peptide libraries such as described in WO0185796, consisting of all possible combinations of amino acids attached to a solid phase support may be used in the present invention. Also transdominant-negative mutant forms of VEGF-receptors (e.g. a transdominant-negative receptor of VEGF-R1) can be used to inhibit the signal transduction of PIGF. Said transdominant-negative mutant forms of VEGF-receptors are fully described in US patent 5,851,999. Also within the scope of the invention is the use of oligoribonucleotide sequences, that include anti-sense RNA and DNA molecules and ribozymes that function to inhibit the translation of VEGFR-1 mRNA or PIGF mRNA. Anti-sense RNA and DNA molecules act to directly block the translation of mRNA by binding to targeted mRNA and preventing protein translation. In regard to antisense DNA, oligodeoxyribonucleotides derived from the translation initiation site, e.g., between - 10 and +10 regions of the VEGFR-1 or PIGF nucleotide sequence, are preferred. Ribozymes are enzymatic RNA molecules capable of catalyzing the specific cleavage of RNA. The mechanism of ribozyme action involves sequence specific hybridization of the ribozyme molecule to complementary target RNA, followed by a endonucleolytic cleavage. Within the scope of the invention are engineered hammerhead motif ribozyme molecules that specifically and efficiently catalyze endonucleolytic cleavage of VEGFR-1 or PIGF sequences. Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for predicted structural features such as secondary structure that may render the oligonucleotide sequence unsuitable. Both anti-sense RNA and DNA molecules and ribozymes of the invention may be prepared by any method known in the art for the synthesis of RNA molecules. These include techniques for chemically synthesizing oligodeoxyribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize anti-sense RNA constitutively or inducibly, depending on the promoter used, can be introduced stably into cell lines.

It should be clear that the therapeutic method of the present invention for the treatment of suppression of adhesion formation can also be used in combination with any other therapy known in the art for the treatment of post operative adhesion formation. The invented compositions for preventing adhesion formation are preferably administered in conjunction with a drug delivery system which maintains an effective concentration of the compound at a site of potential adhesion formation during the perioperative interval. These inhibitors for use in treatment of suppressing and/or minimising adhesion formation can be administered as the primary therapeutic agent or can be co-administered with one or more additional therapeutic agents.

The term 'medicament to treat' relates to a composition comprising molecules as described above and a pharmaceutically acceptable carrier or excipient (both terms can be used interchangeably) to treat diseases as indicated above. Suitable carriers or excipients known to the skilled man are saline, Ringer's solution, dextrose solution, Hank's solution, fixed oils, ethyl oleate, 5% dextrose in saline, substances that enhance isotonicity and chemical stability, buffers and preservatives. Other suitable carriers include any carrier that does not itself induce the production of antibodies harmful to the individual receiving the composition such as proteins, polysaccharides, polylactic acids, polyglycolic acids, polymeric amino acids and amino acid copolymers. The 'medicament' may be administered by any suitable method within the knowledge of the skilled man. The preferred route of administration is parenterally. In parental administration, the medicament of this invention will be formulated in a unit dosage injectable form such as a solution, suspension or emulsion, in association with the pharmaceutically acceptable excipients as defined above. However, the dosage and mode of administration will depend on the individual. Generally, the medicament is administered so that the protein, polypeptide, peptide of the present invention is given at a dose between 1 *µ*g/kg and 10 mg/kg, more preferably between 10 *µ*g/kg and 5 mg/kg, most preferably between 0.1 and 2 mg/kg. Preferably, it is given as a bolus dose. Continuous infusion may also be used and includes continuous subcutaneous delivery via an osmotic minipump. If so, the medicament may be infused at a dose between 5 and 20 *µ*g/kg/minute, more preferably between 7 and 15 *µ*g/kg/minute.

Another aspect of administration for treatment is the use of gene therapy to deliver the above mentioned anti-sense gene or functional parts of the *PIGF* gene or a ribozyme directed against the PIGF, mRNA or a functional part thereof. Gene therapy means the treatment by the delivery of therapeutic nucleic acids to patient's cells. This is extensively reviewed in Lever and Goodfellow 1995; Br. Med Bull.,51, 1-242; Culver 1995; Ledley, F.D. 1995. Hum. Gene Ther. 6, 1129. To achieve gene therapy there must be a method of delivering genes to the patient's cells and additional methods to ensure the effective production of any therapeutic genes. There are two general approaches to achieve gene delivery; these are non-viral delivery and virus-mediated gene delivery.

In a particular embodiment short interference RNA molecules (siRNA) can be used for the manufacture of a medicament for treatment of adhesion formation. Said interference RNA molecules can be generated based on the genetic sequences of PIGF. RNA interference (RNAi) is based on the degradation of particular target sequences by the design of short interference RNA oligo's (siRNA) which recognize the target sequence (here PIGF) and subsequently trigger their degradation by a poorly understood pathway. In general siRNA duplexes are shorter than 30 nucleotides, because longer stretches of dsRNA activate the PKR pathway in mammalian cells which results in a global a-specific shut-down of protein synthesis. The preparation and gene therapy vectors for the intracellular expression of siRNAs duplexes is disclosed in WO0244321.

In another particular embodiment RNA aptamers can be used for the manufacture of a medicament for treatment of adhesion formation. Said RNA aptamers can be generated against PIGF. Recently, RNA aptamers have been used as therapeutic reagents for their ability to disrupt protein function. Selection of aptamers in vitro allows rapid isolation of extremely rare RNAs that have high specificity and affinity for specific proteins. Exemplary RNA aptamers are described in U.S. Pat. No. 5,270,163 to Gold et al., Ellington and Szostak, "In vitro Selection of RNA Molecules That Bind Specific Ligands," Nature 346:818-822 (1990), and Tuerk and Gold, "Systematic Evolution of Ligands by Exponential Enrichment: RNA Ligands to Bacteriophage T4 DNA Polymerase," Science 249:505-510 (1990). Unlike antisense compounds, whose targets are one dimensional lattices, RNA aptamers can bind to the three dimensional surfaces of a protein. Moreover, RNA aptamers can frequently discriminate finely among discrete functional sites of a protein (Gold et al., "Diversity of Oligonucleotide Functions," Annu. Rev. Biochem. 64:763-797 (1995)). As research and therapeutic reagents, aptamers not only have the combined advantages of antibodies and small molecular mass drugs, but in vivo production of RNA aptamers also can be controlled genetically Such RNA expressing genes are usually smaller than protein-coding genes and can be inserted easily into gene therapy vectors.

The invention provides a method for treatment of adhesion formation by administering a compound that inhibits the expression and/or activity of a hypoxia-induced gene wherein said hypoxia induced gene is placental growth factor. Said compound that inhibits the expression and/or activity of placental growth factor is preferably selected from the list consisting of a nucleotide (antisense, siRNA, RNA aptamer), a small molecule, an antibody, a ribozyme, a transdominant receptor, a tetrameric peptide.

The following examples more fully illustrate preferred features of the invention, but are not intended to limit the invention in any way. All of the starting materials and reagents disclosed below are known to those skilled in the art, and are available commercially or can be prepared using well-known techniques.

### Examples and comparative examples

### 1. The role of hypoxia inducible factors and placental growth factor in adhesion formation

### 1.1 Rabbit models and experiments on pneumoperitoneum induced adhesion formation

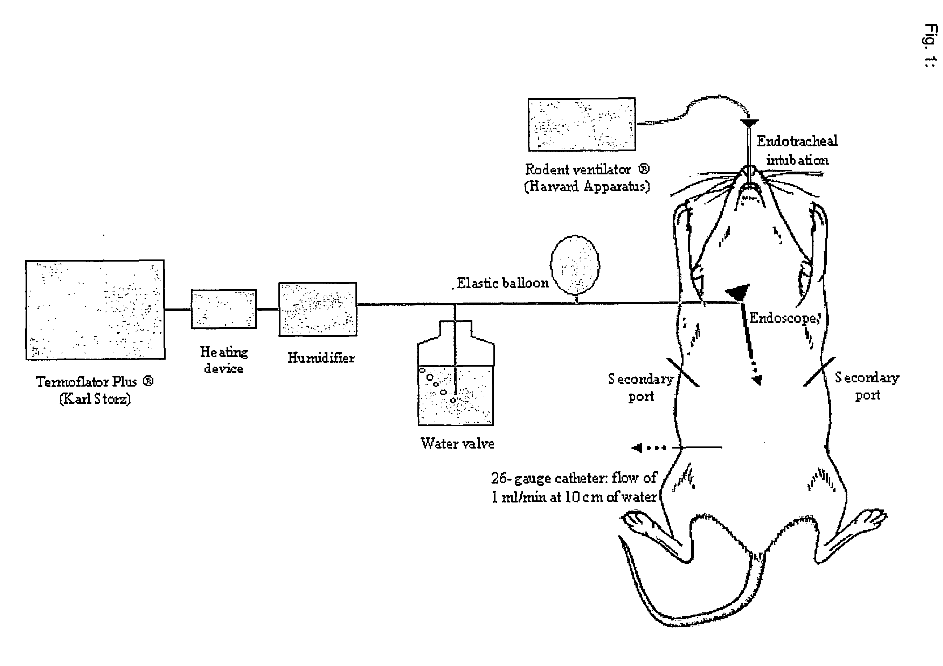

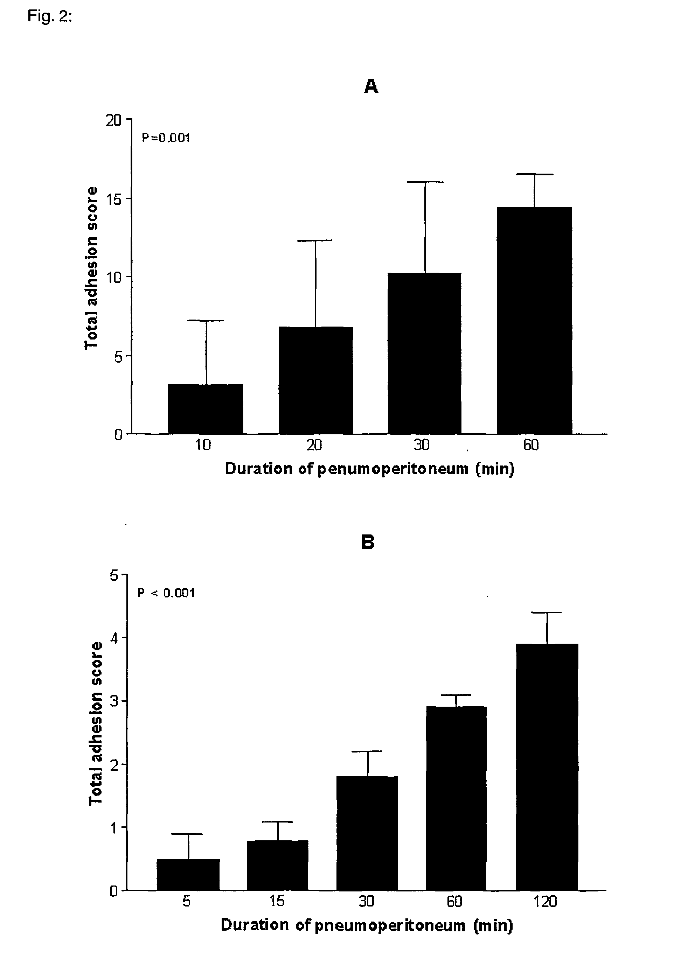

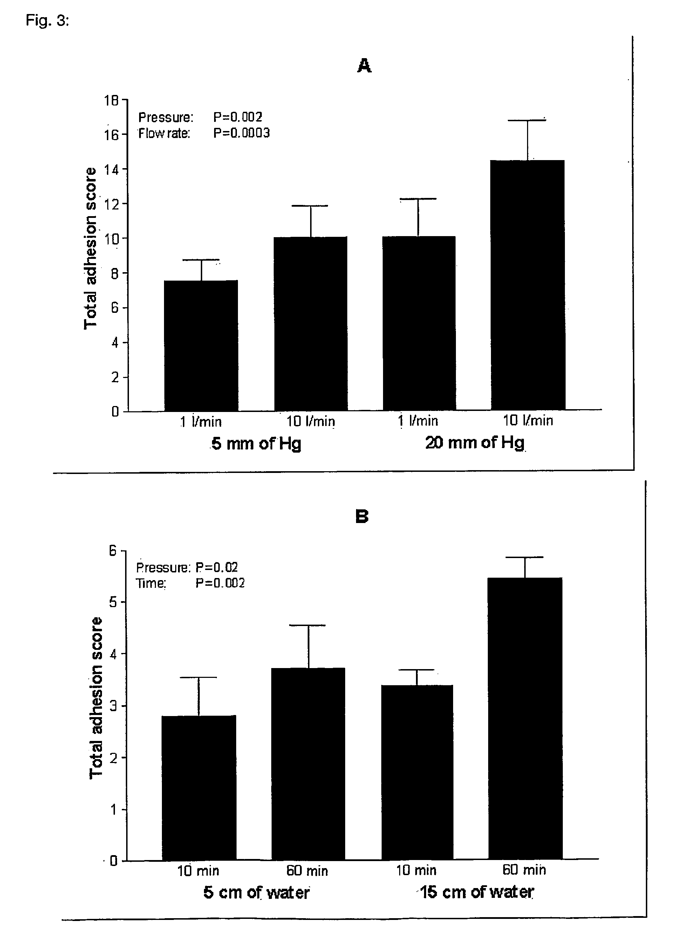

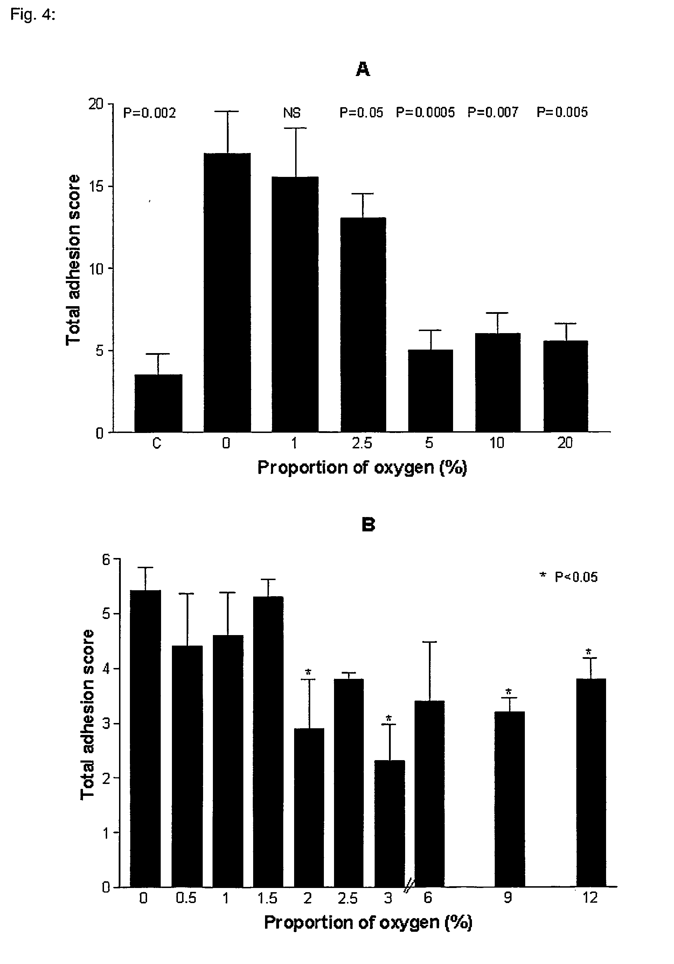

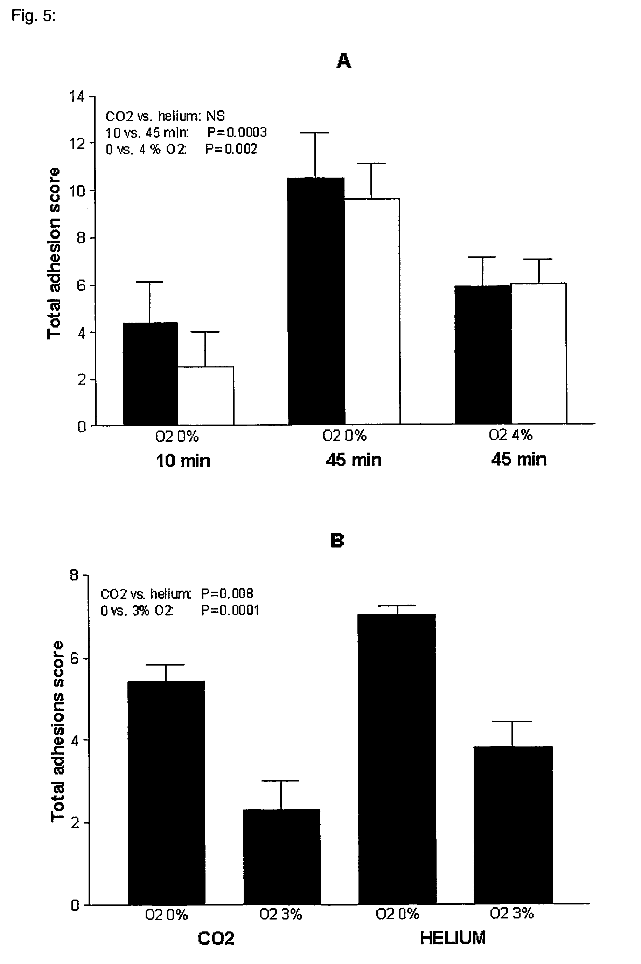

In adult, female, New Zealand, white rabbits adhesions were induced and evaluated during laparoscopy. For the pneumoperitoneum 1 or eventually 2 insufflators (Termoflator®, Karl Storz, Germany) were used, 1 for CO2 or helium and 1 for oxygen. The outputs of both insufflators were connected in a mixing chamber to obtain a homogeneous gas mixture. The gas was heated at 37° C (Optitherm^{®}, Karl Storz, Germany) and humidified (Dräger, Germany) to reduce the known effects of hypothermia (Ott DE: J Laparoendosc Surg 1:183-186, 1991; Ott DE: J Laparoendosc Surg 1:127-131, 1991 and Puttick MI, Scott-Coombes DM, Dye J, Nduka CC, Menzies-Gow NM, Mansfield AO, Darzi A: Surg Endosc 13:572-575, 1999) and desiccation (Ryan GB, Grobety J, Majno G: Mesothelial injury and recovery. Am J Pathol 71:93-112, 1973). The intermittent delivery of gas by an insufflator induces small variations of pressure. They are crucial in small animals because over pressure can became lethal, and since a constant pressure is essential when the effect of insufflation pressure is the aim of the investigation. Therefore a water valve with a free escape of gas was used. The pressure setting in the insufflator was slightly higher than in the water valve knowing that all excess of gas would escape freely avoiding over pressures and maintaining a constant and uniform insufflation pressure. In order to maintain the predefined concentration of insufflation gas a continuous flow rate through the animal, using a 26-gauge catheter, was introduced (Fig. 1).The 1^{st} laparoscopic port (12 mm), for insufflation and the endoscope, was inserted caudal to the xyphoides by open laparoscopy. After the establishment of the pneumoperitoneum the secondary ports (5 mm) were introduced under direct laparoscopic vision. During a first surgery, standardised opposing lesions in uterine horns and pelvic sidewalls were performed with scissors or by bipolar coagulation or by CO₂ laser. After 7 days adhesions were scored during a second surgery assessing extent, type and tenacity. In the first study an inexperienced laparoscopic surgeon performed 50 consecutive surgeries to induce mechanical and bipolar lesions using CO₂ pneumoperitoneum at 5 mm of Hg. The pneumoperitoneum was stopped immediately at the end of the surgical procedure. Duration of surgery decreased (Spearman) from 12 ± 2 min in the first 10 surgeries to 8 ± 1 min in the last 10 (P=0.0001). Simultaneously, total adhesion score decreased (Spearman) from 10 ± 0.8 in the first 10 surgeries to 4.6 ± 0.5 in the last 10 (P=0.002). Duration of surgery and surgeon's experience, assessed by the consecutive number of surgery, however, correlated so strongly that the effect of both could not be separated. It thus was not possible to know the contribution of inexperience of the surgeon and/or duration of surgery upon adhesion formation (Ordonez JL, Dominguez J, Evrard V, Koninckx PR: Hum Reprod 12:2654-2657, 1997). Therefore the effect of the duration of CO₂ pneumoperitoneum was evaluated. Laser and bipolar lesions were performed in 5-6 min and the pneumoperitoneum was maintained for 10, 20, 30 and 60 min. Total adhesion score increased (Wilcoxon) from 3.2 ± 1.9 after 10 min to 6.8 ± 2.4, 10.4 ± 2.6, and 14.4 ± 0.9 after 20, 30 and 60 min respectively (P=0.001) (Fig. 2 A). Similar effects were observed for extent, type and tenacity adhesions scores. These data indicated that CO₂ pneumoperitoneum was a co-factor in adhesion formation. In order to evaluate whether this was mediated by changes in pH or by mesothelial hypoxia, the effect of insufflation pressure, addition of oxygen and of helium, as insufflation gas, were investigated. In intubated rabbits bipolar and laser lesions were performed and CO2 pneumoperitoneum was maintained during 30 min at 5 and 20 mm of Hg to evaluate the effect of insufflation pressure. Since in vitro studies showed more desiccation with higher flow rates (Yesildaglar N, Koninckx PR: Hum Reprod 15:687-691, 2000 and Yesildaglar N, Ordonez JL, Laermans I, Koninckx PR: Hum Reprod 14:55-59, 1999) a flow of 1 and 10 l/min through the animal was used to evaluate the effect of desiccation. Adhesion formation increased (two-way analysis of variance) with a higher insufflation pressure (P=0.002) and with a higher flow rate (P=0.0003) Yesildaglar N, Koninckx PR: Hum Reprod 15:687-691, 2000) (fig. 3 A). Since higher insufflation pressures increases the compression of the capillary flow, these results are consistent with the finding that mesothelial hypoxia plays a key role in adhesion formation suggesting, at the same time, a role for desiccation upon adhesion formation. The addition of oxygen to the CO2 pneumoperitoneum was evaluated using a mixture of CO₂ and oxygen at different proportions. Laser lesions were performed and pneumoperitoneum with 0, 1, 2.5, 5, 10 and 20% of oxygen was maintained for 1 hour. Adhesion formation decreased (Wilcoxon) by adding oxygen and a maximal effect was observed at 5% of oxygen (P=0.0005) (Fig. 4 A). To rule out the effect of acidosis an inert gas, such as helium, was used. 100% CO₂ and helium for 10 and 45 min were used to evaluate the effect of insufflation gas and duration of pneumoperitoneum. Similarly, CO₂ and helium with 0 and 4% of oxygen were used to evaluate the effect of insufflation gas and addition of oxygen. By two-way analysis of variance, adhesion formation increased with duration of CO₂ and helium pneumoperitoneum (P=0.0003) and decreased with the addition of oxygen (P=0.002) whereas no differences were found between CO₂ and helium (Molinas CR, Koninckx PR: Hum Reprod 15:1758-1763, 2000) (Fig. 5 A). Since mesothelial hypoxia should be reduced by oxygen and should not be influenced by the kind of gas and since it is well known that both systemic (Fernandez-Cruz L, Saenz A, Taura P, Sabater L, Astudillo E, Fontanals J: Helium and carbon dioxide pneumoperitoneum in patients with pheochromocytoma undergoing laparoscopic adrenalectomy. World J Surg 22:1250-1255, 1998; Fitzgerald SD, Andrus CH, Baudendistel LJ, Dahms TE, Kaminski DL:. Am J Surg 163:186-190, 1992; Fleming RY, Dougherty TB, Feig BW: Surg Endosc 11:230-234, 1997 and Junghans T, Bohm B, Grundel K, Schwenk W: Arch Surg 132:272-278, 1997) and local (Kuntz C, Wunsch A, Bodeker C, Bay F, Rosch R, Windeler J, Herfarth C: Surg Endosc 14:367-371, 2000 and

West MA, Hackam DJ, Baker J, Rodriguez JL, Bellingham J, Rotstein OD: Ann Surg 226:179-190, 1997) acidosis is caused by CO₂ but not by helium, these results strongly support the hypothesis that mesothelial hypoxia is a co-factor in adhesion formation.

### 1.2 The Mouse model and experiments on pneumoperitoneum induced adhesion formation

In adult, female, NMRI (Naval Medical Research Institute) mice adhesions were induced by laparoscopy and evaluated by laparotomy under microscopic view in order to assess similar variables than in the rabbit model. Since most insufflators have an intermittent delivery of gas, an elastic balloon was incorporated to the previously described set up to dampen pressure changes that are crucial for these small animals (Fig. 1). The endoscope with an outer sheath for insufflation (total diameter 3.2 mm) was introduced caudal to the xyphoides by open laparoscopy. Survival after different insufflation pressures and duration of pneumoperitoneum was evaluated in non-intubated and intubated mice. In non-intubated mice pneumoperitoneum was maintained for 10 min at 2.5, 5, 7.5, 10 and 15 cm of water and the survival was 100, 100, 95, 85 and 60% respectively whereas no mortality was found when pneumoperitoneum was maintained up to 120 min at 2.5 cm of water (Yesildaglar N, Ordonez JL, Laermans I, Koninckx PR: Hum Reprod 14:55-59, 1999). In intubated mice pneumoperitoneum was maintained for 60 min at 5, 10, 15, 20, 25 and 30 cm of water and the survival was 100, 100, 100, 100, 90 and 90% respectively whereas no mortality was found when pneumoperitoneum was maintained up to 180 min at 15 cm of water (Molinas CR, Mynbaev O, Pauwels A, Novak P, Koninckx PR:. Fertil Steril 76:560-567, 2001). For the induction of adhesions, after the establishment of the pneumoperitoneum, the 2^{nd} and 3^{rd} ports (14-gauge catheters) were introduced in the left and right flanks under laparoscopic vision. Standardised, opposing, linear lesions in the anti-mesenteric border of uterine horns and in the pelvic sidewalls were performed by monopolar coagulation. After 7 or 28 days adhesions were evaluated by scoring extent, type and tenacity and by a quantitative measurement. In non-intubated mice CO₂ pneumoperitoneum at 2.5 cm of water was maintained for 5, 15, 30, 60 and 120 min to evaluate the effect of duration of pneumoperitoneum. Adhesion formation increased progressively with duration of pneumoperitoneum (Fig. 2 B). To confirm this effect and to evaluate the effect of desiccation due to the flow rate though the animal, pneumoperitoneum was maintained for 5 and 60 min without flow and for 60 min with 1 and 10 ml/min of flow rate. Adhesion formation increased (Anova) with duration of pneumoperitoneum (P<0.001) and with higher flow rates (P<0.001) confirming the observations in rabbits (Yesildaglar N, Ordonez JL, Laermans I, Koninckx PR: Hum Reprod 14:55-59, 1999). In intubated mice CO₂ pneumoperitoneum was maintained for 10 and 60 min at 5 and 15 cm of water to evaluate the effect of duration of pneumoperitoneum and insufflation pressure. The effect of oxygen was assessed in detail adding 0.5, 1, 1.5, 2, 2.5, 3, 6, 9 and 12% of oxygen to CO₂ pneumoperitoneum. Additionally CO₂ and helium pneumoperitoneum with 0 and 3% of oxygen were used to evaluate the effect of addition of oxygen to different insufflation gases. The mixture of CO₂ or helium with oxygen was done with the Thermoflator Plus^{®} (Karl Storz, Germany), an insufflator used in this study for the first time and developed on base of the promissory results obtained in the rabbit model. Adhesion formation increased with duration of CO₂ pneumoperitoneum and with high insufflation pressure (Fig. 3 B) and decreased with the addition of oxygen (Fig. 4 B). Indeed, half maximal reduction of adhesions was found at around 1.5% of oxygen whereas a maximal response required only 2-3% of oxygen. The addition of oxygen to CO₂ and helium pneumoperitoneum had similar effect in the reduction of adhesions (Fig. 5 B) (Molinas CR, Mynbaev O, Pauwels A, Novak P, Koninckx PR: Fertil Steril 76:560-567, 200). These data confirmed the observations in rabbits and support the hypothesis of mesothelial hypoxia as a driving mechanism. In the rabbit and mouse model it was observed that (i) the postoperative adhesion formation increases with the duration and the pressure of the CO₂ pneumoperitoneum. (ii) the increase in postoperative adhesions caused by CO₂ pneumoperitoneum can be prevented, at least partially, by using a mixture of CO₂ and oxygen. (iii) similar effects upon postoperative adhesion formation are observed when Helium instead of CO₂ is used for the pneumoperitoneum. Adhesions increase with the duration of the pneumoperitoneum, and decrease after the addition of oxygen.

### 1.3 HIF and PIGF mouse models

PIGF -/- has been detailed in lyer, N.V. et al Genes Dev 12, 149-62 (1998); Carmeliet, P. et al Nature 394, 485-90 (1998); Ryan, H.E.., Lo, J. & Johnson, R.S. Embo J. 17, 3005-15 (1998) and Carmeliet, P. et al. Nat. Med. 7, 575-83 (2001).

The material and methods of HIF 1α+/+ and HIF 1α+/- (50% Swiss, 50 % 129) mice and of HIF 2α+/+ and HIF 2α+/- (87.5% Swiss, 12.5% 129) mice has been detailed in lyer, N.V. et al Genes Dev 12, 149-62 (1998); Carmeliet, P. et al Nature 394, 485-90 (1998);

Ryan, H.E.., Lo, J. & Johnson, R.S. Embo J. 17, 3005-15 (1998) and Carmeliet, P. et al. Nat. Med. 7, 575-83 (2001).

### 1.4 Influence of HIF on post operative adhesion formation

A series of experiments in wild type and knock out mice were performed on transgenic mice for HIF, such as HIF 1α+/-, HIF 2α+/- in our laparoscopic mouse model. Adhesions were induced in uterine horns and pelvic sidewalls by linear monopolar and bipolar lesions of 10 watts. In all experiments the pneumoperitoneum was maintained for 10 min to evaluate the *basal adhesions* and for 60 min to evaluate the *pneumoperitoneum-enhanced adhesions.* Pure CO₂ at 20 cm of water (15 mm Hg) of insufflation pressure was used. After 7 days, adhesions were scored quantitatively and qualitatively by laparotomy.

### 1.4.1 HIF 1α

The experiment was performed in HIF 1α+/+ and HIF 1α+/- (50% Swiss, 50 % 129) mice. In HIF 1α+/+ mice adhesion formation increased with duration of pneumoperitoneum whereas in HIF 1α+/- mice a slight reduction in basal adhesions was found with no further increase after pneumoperitoneum. In HIF 1α+/+ mice the proportion of adhesions increased from 12 ± 2% after 10 min (n=5) to 19 ± 4% after 60 min (n=5) of pneumoperitoneum. In HIF 1α+/- mice, the proportion of adhesions were only 4 ± 0.2% after 10 min (n=5) and 6 ± 4% after 60 min of pneumoperitoneum (n=5). Similar results were observed for extent, type, tenacity and total adhesion scores.

### 1.4.2 HIF 2α

The experiment was performed in HIF 2α+/+ and HIF 2α+/- (87.5% Swiss, 12.5% 129) mice. In HIF 2α+/+ mice, adhesion formation increased with duration of pneumoperitoneum whereas in HIF 2α+/- mice a reduction in basal adhesions was found with no further pneumoperitoneum enhancement. In HIF 2α+/+ mice the proportion of adhesions increased from 26 ± 7% after 10 min (n=3) to 36 ± 7% after 60 min (n=3) of pneumoperitoneum. In HIF 2α+/- mice the proportion of adhesions were only 9 ± 3% after 10 min (n=5) and 14 ± 4.6% after 60 min (n=5) of pneumoperitoneum. Similar results were observed for extent, type, tenacity and total adhesion scores.

### 1.5 Influence of PIGF inhibitors on adhesion formation

A series of experiments were performed in wild type and knock-out PIGF mice in our laparoscopic mouse model. Adhesions were induced in uterine horns and pelvic sidewalls by linear monopolar and bipolar lesions of 10 watts. In all experiments the pneumoperitoneum was maintained for 10 min to evaluate the *basal adhesions* and for 60 min to evaluate the *pneumoperitoneum-enhanced adhesions.* Pure CO₂ at 20 cm of water (15 mm Hg) of insufflation pressure was used. After 7 days, adhesions were scored quantitatively and qualitatively by laparotomy.

The experiment was performed in PIGF+/+ and PIGF-/- (50% Swiss, 50 % 129) mice. In PIGF+/+ mice adhesion formation increased with duration of pneumoperitoneum whereas in PIGF-/- mice a slight reduction in basal adhesions was found with no further increase after pneumoperitoneum. In PIGF+/+ mice the proportion of adhesions increased from 7 ± 3% after 10 min (n=5) to 26 ± 5% after 60 min (n=5) of pneumoperitoneum. In PIGF-/mice, the proportion of adhesions were only 5 ± 2% after 10 min (n=5) and 3 ± 1 % after 60 min of pneumoperitoneum (n=5). Similar results were observed for extent, type, tenacity and total adhesion scores.

### 1.5.1 Antibodies against PIGF

The experiment was performed 100% Swiss mice. All animals received every 2 days (days 0, 2, 4 and 6) an introperitoneal injection (20µg/mg dissolved in 200 µl) of one of the following immunoglobulins: mouse IgG (group I), mouse anti-PIGF (non neutralising: PLGE1G11) (group II), mouse anti PIGF (neutralising: PL17A10F12) (group III), mouse anti PIGF (neutralising: PL5D11 D4) (group IV) and mouse anti PIGF (neutralising: PLGH12G5) (group V). In groups I and II the proportion of adhesions increased from 21 ± 3 % (n=5) and 16 ± 4% (n=5) after 10 min to 44 ± 7 % (n=5) and 39 ± 7% (n=5) after 60 min of pneumoperitoneum respectively. In group III a reduction in basal adhesions was found with no further pneumoperitoneum enhancement. The proportion of adhesions was 11 ± 3% after 10 min (n=5) and 12 ± 5% after 60 min (n=5) of pneumoperitoneum. In group IV basal adhesions decrease significantly but a pneumoperitoneum enhancement was observed. The proportion of adhesions was 4 ± 2% after 10 min (n=5) and 11 ± 4% after 60 min (n=5) of pneumoperitoneum. In group V no significant differences with groups I and II were found. The proportion of adhesions was 19 ± 4% after 10 min (n=5) and 34 ± 7 % after 60 min (n=5) of pneumoperitoneum. In all groups similar results were observed for extent, type, tenacity and total adhesion scores.

### 2. The role of vascular endothelial growth factor-B in adhesion formation

In the VEGF-B study, a laparoscopic mouse model permitting to evaluate basal adhesion formation and pneumoperitoneum-enhanced adhesion formation was used. We recognize that the so-called "basal adhesions" not only result from the peritoneal lesion inflicted with the electrocautery but also from the effect of the CO₂ pneumoperitoneum that was present albeit for 10 minutes only. "Basal adhesions" without any additional effect of CO₂ pneumoperitoneum would require the shortest duration possible, the minimum insufflation pressure and some 3% of oxygen added to the CO₂ pneumoperitoneum, since adhesion formation decreases with shorter duration, lower pressure and with the addition of oxygen *(Ordonez JL, Dominguez J, Evrard V, Koninckx PR. The effect of training and duration of surgery on adhesion formation in the rabbit model. Hum Reprod 1997;12:2654-7 - Yesildaglar N, Koninckx PR. Adhesion formation in intubated rabbits increases with high insufflation pressure during endoscopic surgery. Hum Reprod 2000;15:687-91 - Molinas CR, Koninckx PR. Hypoxaemia induced by CO(2) or helium pneumoperitoneum is a co-factor in adhesion formation in rabbits. Hum Reprod 2000;15:1758-63 - Molinas CR, Mynbaev O, Pauwels A, Novak P, Koninckx PR. Peritoneal mesothelial hypoxia during pneumoperitoneum is a cofactor in adhesion formation in a laparoscopic mouse model. Fertil Steril 2001;76:560-7).* In these experiments, for the evaluation of basal adhesions, the pneumoperitoneum was maintained for the minimum time required to perform the lesions (standardized at 10 minutes). We used, however, 100% CO₂ at 20 cm H₂O because a lower pressure and the addition of oxygen, although theoretically better, would introduce additional variables, such as pressure and oxygen. Doing both controls was not feasible because of limited availability of transgenic animals.

In this experiment twenty 100% C57BI/6J wild-type mice (VEGF-B^{+/+}) (female, 10-12 weeks old animals weighing 30-40 g) and transgenic mice deficient for VEGF-B (VEGF-B^{-/-}) were used. As a control fifty 100% Swiss wild-type mice were used. VEGF-B^{-/-} and wild-type mice were obtained from the Ludwig Institute for Cancer Research, Stockholm Branch, Sweden. The transgenic mice were generated as described Aase K. et al (2002) Circulation 104: 358-64. The animals were kept under standard laboratory conditions (temperature 20°-22°C, relative humidity 50-60%, 14 hours light and 10 hours dark) at the animals' facilities of the KUL. They were fed with a standard laboratory diet (Muracon.G, Carsil Quality, Turnhout, Belgium) with free access to food and water at anytime. The study was approved by the Institutional Review Animal Care Committee.

### 2.1 Laparoscopic surgery for induction of intra peritoneal adhesions

Laparoscopy and induction of adhesions were performed as described *(Molinas CR, Mynbaev O, Pauwels A, Novak P, Koninckx PR. Peritoneal mesothelial hypoxia during pneumoperitoneum is a cofactor in adhesion formation in a laparoscopic mouse model. Fertil Steril 2001;76:560-7).* A 3.5-mm midline incision was performed caudal to the xyphoides appendix and a 2-mm endoscope with a 3.3-mm external sheath for insufflation (Karl Storz, Tüttlingen, Germany) was introduced into the abdominal cavity. The endoscope, connected to a video camera (Karl Storz, Tüttlingen, Germany) and light source (Karl Storz, Tüttlingen, Germany), was secured in a holder. Because the mouse abdominal wall is very thin, a variable gas leakage, and thus a variable flow, through the abdomen occurred. Therefore, the incision was closed gas tight around the endoscope with 6/0 polypropylene suture (Prolene, Ethicon, Johnson and Johnson Intl, Brussels, Belgium). For the pneumoperitoneum, the gas was insufflated through the main port with the Thermoflator Plus (Karl Storz, Tüttlingen, Germany) using heated (37° C; Optitherm, Karl Storz, Tüttlingen, Germany) and humidified (Aquapor, Dräger, Lübeck, Germany) CO₂ as insufflation gas. An insufflation pressure of 17 mm Hg and a flow rate of 1.5 l/minutes together with a water valve and an elastic balloon were used to ascertain a continuous insufflation pressure of 20 cm H₂O (≅ 15 mm Hg). The water valve and the balloon are necessary to adapt the flow rate to a mouse and to dampen the pressure changes during insufflation. Indeed, any excess of CO₂ freely escapes from the water valve, whereas pressure is maintained accurately in the water valve and pressure changes are minimized. Since the peritoneum has a large surface and high exchange capacity, theoretically some oxygen could diffuse from the circulation to the abdominal cavity. In order to ascertain continuously a constant 100% CO₂ concentration in the abdominal cavity the gas was continuously replaced. This was achieved by inserting a 26-gauge needle (BD Plastipak, Becton Dickinson, Madrid, Spain) through the abdominal wall, giving a continuous flow through the abdominal cavity of 10 ml/minutes at 20 cm H₂O. After the establishment of the CO₂ pneumoperitoneum, two 14-gauge catheters (Insyte-W, Vialon, Becton Dickinson, Madrid, Spain) were inserted under laparoscopic vision in both right and left flank for the working instruments. The uterus was grasped in the midline with a 1.5-mm grasper and standardized 10-mm x 1.6-mm lesions were performed in the antimesenteric border of both right and left uterine horns by monopolar or bipolar coagulation (10 watts, standard coagulation mode, 10 seconds) (Autocon 350, Karl Storz, Tüttlingen, Germany). In addition, identical lesions were made in right and left pelvic sidewalls. The type of lesion in each side was randomly determined. Monopolar coagulation was performed with a homemade 1.6-mm ball electrode whereas bipolar coagulation was performed with a 1.6-mm probe (Bicap, Circon Corporation, Santa Barbara, CA, USA). Although in all previous experiments monopolar lesions induced more adhesions than bipolar lesions, the experimental design to induce adhesions is kept constant in order to facilitate comparison between the experiments and to detect eventual differences in the mechanism of adhesion formation between both lesions. In order to evaluate postoperative "basal adhesion formation" and "pneumoperitoneum-enhanced adhesion formation", the pneumoperitoneum was maintained for the minimum time needed to induce the peritoneal lesions (standardized at 10 minutes) or for a longer period (60 minutes), respectively. The secondary ports were removed after finalizing the peritoneal lesions and the incisions were closed. The first incision was closed at the end of the surgery. All incisions were closed in a single layer with 6/0 polypropylene suture (Prolene, Ethicon, Johnson and Johnson Intl, Brussels, Belgium).

### 2.2 Experimental design

The experiments were performed using block randomization by days. Therefore, one block of mice, comprising one animal of each group, was operated during the same day. Within a block the animals were operated in random order. In the experiment (n=20), basal adhesions and pneumoperitoneum-enhanced adhesions were assessed in VEGF-B^{+/+} mice (n=5 and n=5, respectively) and VEGF-B^{-/-} mice (n=5 and n=5, respectively).

### 2.3 Results

All animals survived the surgical procedures and all of them were available for adhesion scoring after seven days. Adhesions only formed between the injured visceral site and the pelvic fat or between the injured parietal site and the pelvic fat. No adhesions were observed at the site of the laparoscopic ports or at other sites. In all experiments monopolar lesions systematically induced more adhesions than bipolar lesions. Since no obvious differences were observed in the regulation of adhesions following monopolar or bipolar lesions, and in order to maximize statistical significance, only the means of both lesions are discussed in this invention. In VEGF-B wild-type mice, pneumoperitoneum enhanced adhesion formation (proportion: P=0.02; type: P=0.04; total: P=0.05). In comparison with VEGF-B wild-type mice, basal adhesions were similar in VEGF-B^{-/-} mice. In VEGF-B^{-/-} mice, pneumoperitoneum did not enhance adhesion formation. Therefore, in comparison with VEGF-B wild-type mice, pneumoperitoneum-enhanced adhesions were obviously lower in VEGF-B^{-/-} mice (proportion: P=0.05; type: P=0.03; total: P=0.05).

This study confirms our previous finding that the pneumoperitoneum is a cofactor in adhesion formation since pneumoperitoneum-enhanced adhesions were observed in all wild-type mice used as control animals *(Ordonez JL, Dominguez J, Evrard V, Koninckx PR. The effect of training and duration of surgery on adhesion formation in the rabbit model. Hum Reprod 1997;12:2654-7 - Yesildaglar N, Koninckx PR. Adhesion formation in intubated rabbits increases with high insufflation pressure during endoscopic surgery. Hum Reprod 2000;15:687-91 - Molinas CR, Koninckx PR. Hypoxaemia induced by CO(2) or helium pneumoperitoneum is a co-factor in adhesion formation in rabbits. Hum Reprod 2000;15:1758-63 - Molinas CR, Mynbaev O, Pauwels A, Novak P, Koninckx PR. Peritoneal mesothelial hypoxia during pneumoperitoneum is a cofactor in adhesion formation in a laparoscopic mouse model. Fertil Steril 2001;76:560-7).* To the best of our knowledge this is the first study demonstrating directly a role of VEGF-B in postoperative adhesion formation. Our results can be explained by postulating that pneumoperitoneum enhances adhesion formation, at least in part, through an up-regulation of VEGF-B. In VEGF-B^{-/-} mice basal adhesions were comparable with those in wild-type mice, suggesting that VEGF-B has no major role in basal adhesion formation. In these VEGF-B^{-/-} mice adhesion formation did not increase following 60 minutes of CO₂ pneumoperitoneum, demonstrating that the mechanism of CO₂ pneumoperitoneum-enhanced adhesions involves VEGF-B, which obviously cannot be up-regulated in these mice.

### 3. Scoring of adhesions

A xyphopubic midline incision and a bilateral subcostal incision were performed and the whole abdominal cavity was explored during laparotomy seven days after the induction of adhesions as described *(Molinas CR, Mynbaev O, Pauwels A, Novak P, Koninckx PR. Peritoneal mesothelial hypoxia during pneumoperitoneum is a cofactor in adhesion formation in a laparoscopic mouse model. Fertil Steril 2001;76:560-7).* After the evaluation of port sites and viscera, the pelvic fat tissue was carefully removed and adhesions were blindly scored under microscopic vision using a qualitative and a quantitative scoring system. In the qualitative scoring system the following characteristics were assessed (modified from Leach) *(Leach RE, Burns JW, Dawe EJ, SmithBarbour MD, Diamond MP. Reduction of postsurgical adhesion formation in the rabbit uterine horn model with use of hyaluronate*/*carboxymethylcellulose gel. Fertil Steril 1998; 69:415-8): extent* (0: no adhesions; 1: 1-25%; 2: 26-50%; 3: 51-75%; 4: 76-100% of the injured surface involved, respectively), *type* (0: no adhesions; 1: filmy; 2: dense; 3: capillaries present), *tenacity* (0: no adhesions; 1: essentially fall apart; 2: require traction; 3: require sharp dissection) and *total* (extent + type + tenacity). In addition, a quantitative scoring system was used as described by Holmdahl *(Holmdahl L, al Jabreen M, Risberg B. Experimental models for quantitative studies on adhesion formation in rats and rabbits. Eur Surg Res 1994;26:248-56).* This system has the advantage to be devoid of any subjective interpretation. It measures the proportion of the lesions covered by adhesions using the following formula: adhesions (%) = (sum of the length of the individual attachments/length of the lesion) x 100. The results are presented as the average of the adhesions formed at the four individual sites (right and left visceral and parietal peritoneum with lesions inflicted by monopolar or bipolar coagulation), which were individually scored.

### 4. Statistics

Statistical analysis was performed with the SAS System (SAS institute, Cary, NC, USA) using the non-parametric Kruskal-Wallis test to compare individual groups and Spearman correlation to evaluate association. All data are presented as the mean ± SE.

### 5. Anesthesia

After the IM anesthesia with pentobarbital (Nembutal, Sanofi Sante Animale, Brussels, Belgium; 0.07 mg/g) the abdomen was shaved and the animal was secured to the table in supine position. Endotracheal intubation was performed with a 22-gauge catheter (Insyte-W, Vialon, Becton Dickinson, Madrid, Spain) by transillumination of the vocal cords as described (Molinas CR, Mynbaev O, Pauwels A, Novak P, Koninckx PR. Peritoneal mesothelial hypoxia during pneumoperitoneum is a cofactor in adhesion formation in a laparoscopic mouse model. Fertil Steril 2001 ;76:560-7). The catheter was connected to a mechanical ventilator (Rodent Ventilator, Harvard Apparatus, Holliston, MA, USA) and the animal was ventilated with room air with a tidal volume of 500 *µ*L and a respiratory rate of 85 strokes/minutes.

## Claims

1. Use of a compound that inhibits the expression and/or activity of a placenta growth factor for the manufacture of a medicament for treatment of adhesion formation.

2. Use of a compound according to claim 1, wherein said compound is selected from the list consisting of a nucleotide, an antibody, a ribozyme, a transdominant receptor, a tetrameric peptide.

3. Use of a compound according to claim 2, wherein said nucleotide is an antisense DNA or RNA, siRNA, or an RNA aptamer.

4. Use according to claim 2, wherein said antibody is a monoclonal antibody specifically directed to PIGF or antigen-binding fragment thereof.

5. Use according to claim 4, wherein said antibody or antibody fragment is humanized.

## Patentansprüche

1. Verwendung einer Verbindung, die die Expression und/oder Aktivität eines Plazenta-Wachstumsfaktors hemmt, zur Herstellung eines Medikamentes zur Behandlung einer Adhäsionsbildung.

2. Verwendung einer Verbindung nach Anspruch 1, wobei die Verbindung aus der Liste ausgewählt ist, die aus einem Nukleotid, einem Antikörper, einem Ribozym, einem transdominanten Rezeptor, einem tetrameren Peptid besteht.

3. Verwendung einer Verbindung nach Anspruch 2, wobei das Nukleotid eine Antisense-DNA oder RNA, siRNA oder ein RNA Aptamer ist.

4. Verwendung nach Anspruch 2, wobei der Antikörper ein monoklonaler Antikörper ist, der spezifisch gegen PIGF oder ein antigen-bindendes Fragment hiervon gerichtet ist.

5. Verwendung nach Anspruch 4, wobei der Antikörper oder das Antikörperfragment humanisiert ist.

## Revendications

1. Utilisation d'un composé qui inhibe l'expression et / ou l'activité d'un facteur de croissance du placenta pour fa fabrication d' un médicament pour le traitement de la formation d' adhérences.

2. Utilisation d'un composé selon la revendication 1, dans laquelle ledit composé est choisi parmi la liste constituée d'un nucléotide, d'un anticorps, d'un ribozyme, d'un récepteur transdominant et d'un peptide tétramère.

3. Utilisation d'un composé selon la revendication 2, dans laquelle ledit nucléotide est un AND ou ARN antisens, un siARN, ou un aptamère ARN.

4. Utilisation selon la revendication 2, dans laquelle ledit anticorps est un anticorps monoclonal spécifiquement dirigé contre PIGF ou un fragment se liant à un antigène de celui-ci.

5. Utilisation selon la revendication 4, dans laquelle ledit anticorps ou fragment d'anticorps est humanisé.


### SEQUENCE LISTING

<110> VIB VZW
<120> Tissue adesion formation control
<130> VIB-033-PCT
<150> GB 0202379.4
   <151> 2002-02-04
<150> GB 0225128.8
   <151> 2002-01-29
<150> GB 0201983.4
   <151> 2002-01-29
<160> 1
<170> PatentIn version 3.1
<210> 1
   <211> 11
   <212> PRT
   <213> Artificial sequence
<220>
   <223> PR11 (a truncated form of PR-39)
   <400> 1
   Arg Arg Arg Pro Arg Pro Pro Tyr Leu Pro Arg
   1               5                   10